# FASCICULE DE BREVET EUROPEEN

(11) **EP 4 405 371 B1**
(45) Date de publication et mention de la délivrance du brevet: **18.11.2026**
(21) Numéro de dépôt: 22793398.3
(22) Date de dépôt: 20.09.2022
(51) Int. Cl.: C07K 7/06, A61K 8/64, A61K 38/08, A61K 38/10, A61P 17/00, A61P 17/02, A61Q 19/08, C07K 7/08, C07K 14/78, A61K 38/00

(54) **PEPTIDES ET COMPOSITIONS PHARMACEUTIQUES ET COSMETIQUES LES CONTENANT**
PEPTIDE UND DIESE ENTHALTENDE PHARMAZEUTISCHE UND KOSMETISCHE ZUSAMMENSETZUNGEN
PEPTIDES AND PHARMACEUTICAL AND COSMETIC COMPOSITIONS COMPRISING THE SAME

(30) Priorité: 20.09.2021 FR 2109849
(43) Date de publication de la demande: 31.07.2024
(73) Titulaire: Laboratoires d'Anjou, 75008 Paris (FR); Centre National de la Recherche Scientifique (CNRS), 75794 Paris Cedex 16 (FR); Université Claude Bernard Lyon 1, 69100 Villeurbanne (FR)
(72) Inventeur: ROUSSELLE, Patricia, 69005 LYON (FR)
(74) Mandataire: Yes My Patent
(86) Numéro de dépôt international: PCT/EP2022/076100
(87) Numéro de publication internationale: WO 2023/041801

(56) Documents cités:
- WO-A1-2006/018551
- WO-A1-2010/103254
- WO-A1-2020/069018
- WO-A2-00/66731

## Description

### DOMAINE DE L'INVENTION

La présente invention se rapporte à de nouveaux peptides ayant le même motif LRIRX₁TYX₂, où X₁ a pour acide aminé A et X₂ a pour acide aminé G ; ou X₁ a pour acide aminé F et X₂ est choisi parmi les acides aminés G ou K. La présente invention concerne également leur utilisation dans le traitement des altérations de la peau d'origines diverses, ainsi que des compositions pharmaceutiques et cosmétiques les comprenant et un procédé cosmétique. Plus particulièrement, le traitement desdites altérations consiste notamment à renforcer la jonction dermo-épidermique, l'adhérence cellule-cellule et/ou l'adhérence cellule-matrice et la migration des cellules au niveau de l'épiderme et à favoriser la réparation de la surface cutanée.

### ETAT DE LA TECHNIQUE

L'altération de la peau la plus commune et la plus naturelle est tout simplement celle due au vieillissement cutané. Le vieillissement cutané est un phénomène complexe qui est dû à de nombreux facteurs intrinsèques (facteurs génétiques) et extrinsèques (tels que le soleil, l'alimentation, l'exposition à la fumée de cigarette...). Cliniquement, on observe l'apparition de rides et de ridules, une perte de l'élasticité cutanée, un relâchement des tissus cutanés et sous-cutanés ainsi qu'une moins bonne cicatrisation.

De nombreuses voies de recherches sont proposées pour lutter contre le vieillissement cutané, parmi lesquelles la protection contre l'environnement (soleil, pollutions...), l'activation de la régénération cellulaire, le renforcement de la matrice extracellulaire (collagène et élastine). Récemment, des études ont montré l'importance de l'adhérence des cellules entre elles d'une part, et de l'adhérence entre les cellules et la matrice extracellulaire d'autre part, dans le processus du vieillissement cutané et donc de la nécessité à les renforcer pour prévenir voire traiter le relâchement de la peau.

Les études dermatologiques récemment tournées vers l'utilisation des peptides en biologie cutanée (séquences dérivées de l'alpha-MSH, certains neuropeptides, peptide RGD...), s'orientent vers la recherche de peptides ayant une activité forte au niveau cutané.

L'industrie cosmétique est également dans l'attente permanente de nouveaux peptides capable d'augmenter l'adhérence des cellules à la matrice extracellulaire et/ou l'adhérence des cellules entre elles.

Les interactions entre les cellules et la matrice extracellulaire (MEC) jouent en effet un rôle important dans le contrôle du comportement cellulaire. Ce contrôle s'effectue par des interactions spécifiques entre les molécules matricielles et les cellules au niveau de récepteurs transmembranaires, principalement de la famille des intégrines, dont le domaine intracellulaire est relié aux constituants du cytosquelette. Ceci permet à la matrice d'assurer la transmission de signaux intracellulaires modulant selon les cas l'adhérence, la migration, la prolifération, la différentiation ou l'apoptose des cellules de l'épiderme. Ce contrôle du comportement cellulaire est crucial au cours du développement mais également lors des remaniements tissulaires.

Selon les molécules constitutives et l'organisation qui en découle, on distingue plusieurs types de MEC, les lames basales étant sans doute les plus spécialisées. Ces dernières sont de fins treillis protéiques continus sur lesquels reposent les différents feuillets cellulaires de l'organisme. Elles ont longtemps été définies comme des structures morphologiques discrètes dont la fonction était limitée au cloisonnement des compartiments tissulaires. C'est seulement au cours des 25 dernières années que, malgré leur faible représentativité et leur grande insolubilité, des avancées significatives ont été réalisées dans l'investigation de leur composition moléculaire et de leurs fonctions. Il apparaît que ces structures jouent un rôle fondamental dans le contrôle du comportement cellulaire, tant au cours du développement embryonnaire que dans le maintien de l'intégrité des phénotypes cellulaires différenciés.

La structure de la peau est composée :
- d'un épithélium de revêtement : l'épiderme,
- d'un tissu conjonctif : le derme (couche épaisse déterminant la morphologie de la peau et contenant les vaisseaux sanguins et lymphatiques, les nerfs), et
- d'un tissu adipeux : l'hypoderme.

Derme et épiderme sont reliés par une structure unique et complexe, la jonction dermo-épidermique (JDE) ou lame basale épidermique. Anatomiquement, elle correspond à la zone comprise entre les cellules basales de l'épiderme et les couches les plus superficielles du derme. C'est une zone d'adhérence qui détermine un cloisonnement entre l'épithélium polarisé et le stroma sous-jacent, assurant le maintien et la cohésion des cellules adjacentes. La JDE, composée exclusivement de MEC, assure deux rôles :
1°) rôle mécanique, son architecture moléculaire unique lui confère une grande stabilité mécanique lui permettant d'assurer l'ancrage solide de l'épiderme ;
2°) rôle biologique, les protéines de la JDE entretiennent avec les cellules basales de l'épiderme des relations étroites et leur transmettent des informations morphogénétiques importantes par l'intermédiaire des récepteurs de la famille des intégrines.

La JDE est aussi un filtre de diffusion vis-à-vis des éléments nutritifs et métaboliques. Elle permet donc la transmission des informations biologiques.

Au cours du vieillissement cutané, la JDE s'aplanit et perd progressivement ses ondulations caractéristiques, ce qui réduit considérablement l'interface épiderme-derme. De plus, les réseaux moléculaires se désorganisent, la charpente protéique se fragilise et l'adhérence des kératinocytes basaux est amoindrie.

En conséquence, la fonction mécanique (soutien) et la fonction biologique (échange d'informations et de molécules) de la JDE sont altérées.

Lorsqu'il y a blessure cutanée, la JDE est endommagée et ses molécules constitutives sont dégradées par des enzymes spécifiques. Dans des conditions favorables, la réépithélialisation épidermique débute quelques heures après le traumatisme. Dès que l'épithélium a recouvert le lit de la plaie, les protéines de la JDE réapparaissent de façon séquentielle et ordonnée.

Avec le vieillissement cutané, chacune de ces étapes se déroule plus lentement. En particulier, il a été observé une diminution de l'expression des protéines d'adhérence matricielle ainsi que de celle des récepteurs membranaires, qui pourrait constituer la cause majeure du retard observé dans le processus de reconstitution de la JDE et de l'extrême fragilité des tissus cicatrisés chez les sujets âgés.

La laminine 5 (LN-5) ou laminine 332 est une protéine spécifiquement exprimée dans les lames basales des épithélia squameux et transitionnels spécialisés comme la JDE de la peau. La LN-5 résulte de l'assemblage hétérotrimérique des sous-unités alpha 3, bêta 3 et gamma 2 et est synthétisée exclusivement par les cellules épithéliales sous la forme d'un précurseur de 460 kDa. Dans les conditions physiologiques, chacune des sous-unités alpha 3 et gamma 2 subit un clivage post-traductionnel extracellulaire des extrémités carboxy-et amino-terminales respectivement, aboutissant à la forme tissulaire mature. Des études indiquent que la totalité de la chaîne gamma 2 précurseur est nécessaire à la sécrétion et à l'intégration de la LN-5 dans la MEC. D'autres études ont montré que la chaîne gamma 2 précurseur est impliquée dans la migration des kératinocytes lors du processus de cicatrisation cutanée.

Le rôle majeur de la LN-5 est souligné par l'existence de pathologies héréditaires ou acquises, résultant d'une anomalie de synthèse et/ou d'expression de l'une de ses sous-unités constitutives. Ces maladies, appelées épidermolyses bulleuses jonctionnelles, conduisent notamment à une fragilité de la JDE de la peau caractérisée par la formation spontanée de bulles épidermiques. La LN-5 joue ainsi un rôle crucial et irremplaçable dans la cohésion épithélio-mésenchymateuse. La LN-5 est porteuse de signaux biologiques déterminants puisqu'elle permet l'adhérence des cellules épithéliales adjacentes par l'intermédiaire des intégrines et induit l'assemblage des structures d'adhérence stable que sont les hémidesmosomes.

La demande de brevet internationale WO 00/66731 au nom de Biostatum décrit la production de LN-5 entière sous forme recombinante dans des cellules eucaryotes (L5r) et documente son activité pour améliorer la cicatrisation, la prolifération et l'adhérence cellulaire. D'autres peptides dérivés de la Laminine-5 sont décrits en US-B1-6294356 (Jones).

Le brevet EP1784423, déposée par la Demanderesse, caractérise l'efficacité d'un peptide de séquence TALRIRATYGEY, séquence présente sur la chaîne gamma 2 de la LN-5, qui induit de façon spécifique l'adhérence des kératinocytes de l'épiderme et d'autres cellules épithéliales.

Il perdure un besoin d'alternatives pour le traitement des altérations de la peau d'origines diverses, telles que décrites ci-dessus, qui plus est ayant une efficacité accrue.

Aujourd'hui, la Demanderesse a identifié, de façon surprenante et inattendue, qu'une quantité efficace d'un peptide consistant en la séquence LRIRX₁TYX₂ où X₁ a pour acide aminé A et X₂ a pour acide aminé G ; ou X₁ a pour acide aminé F et X₂ est choisi parmi les acides aminés G ou K induit de façon spécifique l'adhérence des kératinocytes de l'épiderme et d'autres cellules épithéliales. Ce peptide permet non seulement d'augmenter l'adhérence des cellules à la MEC mais également d'augmenter la migration des kératinocytes. De par sa petite taille et sa grande stabilité, le peptide présente toutes les caractéristiques requises pour traverser convenablement l'épiderme et atteindre sa cible, la JDE, et interagir avec les kératinocytes basaux et transmettre des signaux d'induction d'adhérence. Son procédé de fabrication par synthèse chimique est simple et applicable à l'échelle industrielle. Il ne fait pas appel à l'utilisation de cultures de cellules d'origine animale, ni à des facteurs de croissance et/ou de sérums ou dérivés de sérums. De petite taille, il sera peu ou pas la cible de dégradations protéolytiques spécifiques et/ou non spécifiques.

Le concept inventif général selon l'invention est l'utilisation d'un peptide consistant en la séquence : LRIRX₁TYX₂, où :
- X₁ a pour acide aminé A et X₂ a pour acide aminé G ; ou
- X₁ a pour acide aminé F et X₂ est choisi parmi les acides aminés G ou K.

C'est ce motif qui induit de façon spécifique les effets mentionnés ci-dessous. Les variantes permettant de cycliser ou de doubler la présence de ce peptide ne sont que des variantes de format de ce motif objet de l'invention, pouvant influencer légèrement son efficacité.

### EXPOSE DE L'INVENTION

Selon un premier aspect, l'invention concerne un peptide consistant en la séquence : LRIRX₁TYX₂, où :
- X₁ a pour acide aminé A et X₂ a pour acide aminé G ; ou
- X₁ a pour acide aminé F et X₂ est choisi parmi les acides aminés G ou K.

Selon un mode de réalisation, ledit peptide présente la séquence suivante : LRIRFTYK (SEQ ID NO : 1)
Selon un mode de réalisation, ledit peptide présente la séquence suivante : LRIRFTYG (SEQ ID NO : 5)
Selon un mode de réalisation, ledit peptide présente la séquence suivante : LRIRATYG (SEQ ID NO : 13)

Selon un deuxième aspect, l'invention concerne un peptide consistant en la séquence : CLRIRX₁TYX₂C, où :
- X₁ a pour acide aminé A et X₂ a pour acide aminé G ; ou
- X₁ a pour acide aminé F et X₂ est choisi parmi les acides aminés G ou K.

L'ajout de ces deux cystéines aux extrémités du peptide permet sa cyclisation.
Selon un mode de réalisation, ledit peptide présente la séquence suivante : CLRIRFTYKC (SEQ ID NO : 2)
Selon un mode de réalisation, ledit peptide présente la séquence suivante : CLRIRFTYGC (SEQ ID NO : 6)
Selon un mode de réalisation, ledit peptide présente la séquence suivante : CLRIRATYGC (SEQ ID NO : 14)

Selon un troisième aspect, l'invention concerne un peptide consistant en la séquence : CSGLRIRX₁TYX₂SGC, où :
- X₁ a pour acide aminé A et X₂ a pour acide aminé G ; ou
- X₁ a pour acide aminé F et X₂ est choisi parmi les acides aminés G ou K.

L'ajout de ces deux cystéines ainsi que des espaceurs Sérine-Glycine aux extrémités du peptide permet sa cyclisation.
Selon un mode de réalisation, ledit peptide présente la séquence suivante : CSGLRIRFTYKSGC (SEQ ID NO : 3)
Selon un mode de réalisation, ledit peptide présente la séquence suivante : CSGLRIRFTYGSGC (SEQ ID NO : 7)
Selon un mode de réalisation, ledit peptide présente la séquence suivante : CSGLRIRATYGSGC (SEQ ID NO : 15)

La présente description décrit en outre un peptide consistant en la séquence LRIRX₁TYX₂SGLRIRX₁TYX₂, où :
- X₁ est choisi parmi les acides aminés A ou F ; et
- X₂ est choisi parmi les acides aminés G ou K.

L'ajout de ces espaceurs Sérine-Glycine permet d'espacer ce peptide qui comprend deux fois le motif du peptide selon l'invention.

Dans un cas, ledit peptide présente la séquence suivante : LRIRFTYKSGLRIRFTYK (SEQ ID NO : 4)

Dans un cas, ledit peptide présente la séquence suivante : LRIRFTYGSGLRIRFTYG (SEQ ID NO : 8)

Dans un cas, ledit peptide présente la séquence suivante : LRIRATYKSGLRIRATYK (SEQ ID NO : 12)

Dans un cas, ledit peptide présente la séquence suivante : LRIRATYGSGLRIRATYG (SEQ ID NO : 16)

La présente description décrit également d'autres peptides résultant d'une ou plusieurs modification(s) des peptides ci-dessus, la suppression ou la substitution d'un ou plusieurs acides aminés, de préférence comme indiqué dans le tableau 1, et/ou d'une oligomérisation, d'une cyclisation ou du repliement des peptides ci-dessus, étant entendu que ces modifications ne diminuent en rien l'activité adhésive du peptide de référence, voire l'améliorent.

**Tableau 1 : Substitutions possibles des acides aminés des peptides selon l'invention**

| **Position des acides aminés** | **SEQ** | **Acides aminés de remplacement possibles** |
|---|---|---|
| 1 | L | L, M, I, V, F, E ou R |
| 2 | R | R, N, E, Q, H, K ou Y |
| 3 | I | I, M, L, V, F, Tou K |
| 4 | R | R, N, E, Q, H, K ou T |
| 5 | X (A ou F) | A, C, S, G, V ou D |
| 6 | T | T, S, P, G, N, D, E, Q, H, ou K |
| 7 | Y | Y, H, F, W, D, I ou Q |
| 8 | X (G ou K) | G, S, T, A ou N |

| | | |
|---|---|---|
| * X pouvant être n'importe quel acide aminé. | | |

Plus particulièrement, l'ajout ou le retrait d'un ou plusieurs acides aminés peut être réalisé du côté carboxy et /ou du coté amino-terminal dudit peptide. La présente description décrit également tout analogue ou dérivé qui résulterait de la greffe d'un motif d'intérêt (molécules naturelles ou synthèse, protéines et/ou sucres) sur ledit peptide. Ainsi que tout fragment dermatologiquement actif du peptide de l'invention, modifié ou non. De manière préférée, les peptides selon l'invention sont obtenus par synthèse chimique.

L'invention a également pour objet l'utilisation desdits peptides selon l'invention comme médicament.

Selon un mode de réalisation, lesdits peptides selon l'invention sont utilisés dans le renforcement de la jonction dermo-épidermique, l'adhérence cellule-cellule et/ou l'adhérence cellule-matrice et la migration des cellules au niveau de l'épiderme.

Comme indiqué précédemment, l'altération de l'épiderme et de la JDE la plus communément observée est celle résultant du vieillissement cutané. Cependant, d'autres altérations de la peau peuvent intervenir indépendamment du vieillissement et peuvent par exemple être induites par certaines pathologies dermatologiques. A titre d'exemple, on peut citer l'eczéma, le psoriasis, le prurit les dermites irritatives, l'héliodermie, les kératoses, les mycoses, l'ichtyose. Outre les symptômes spécifiques à chacune de ces pathologies, la peau subit des altérations auxquelles se propose de remédier la présente invention à titre de complément thérapeutique. Il est clair que la présente invention n'a pas pour but le traitement de telles pathologies mais de restaurer la JDE endommagée et l'adhérence cellulaire dans ces pathologies et la régénération de l'épiderme en favorisant la migration des kératinocytes.

Ainsi la présente invention permet la réparation, la régénération et/ou la restructuration de la peau.

La peau peut également être fragilisée par des traitements cosmétiques ou thérapeutiques de la peau qui, tout en traitant la pathologie visée, génèrent des effets secondaires sur la peau qu'il convient de traiter indépendamment de la pathologie elle-même. C'est le cas notamment des traitements de l'acné, des traitements par puvathérapie, des interventions chirurgicales (dermatologiques ou autres), des traitements de la peau par laser, de la dermabrasion, des peelings ou des traitements de cancers par radiothérapie.

Selon un mode de réalisation, lesdits peptides selon l'invention sont utilisés dans le traitement des altérations de la peau induites par des pathologies dermatologiques.

Selon un mode de réalisation, lesdits peptides selon l'invention sont utilisés dans le traitement des altérations de la peau fragilisée par des traitements cosmétiques ou thérapeutiques.

Selon un mode de réalisation, lesdits peptides selon l'invention sont utilisés dans le traitement curatif ou préventif du vieillissement cutané tel que les rides, le relâchement, la perte d'élasticité, de la moins bonne cicatrisation, des cicatrices chéloïdes, hypertrophiques ou atrophiques, de la xérose sénile, des modifications du système pigmentaire de la peau, de l'appauvrissement du réseau vasculaire de la peau, de l'irrégularité du grain de peau, de l'atrophie cutanée et de l'altération des phanères.

En effet, les susdites pathologies, fragilisations de la peau et traitements divers génèrent sur la peau des altérations telles que la diminution de l'adhérence de l'épiderme et de la cohésion épidermique, ou encore une moins bonne restructuration de la surface cutanée.

Selon un autre aspect, l'invention concerne une composition pharmaceutique comprenant au moins un peptide selon l'invention.

De manière préférée, ladite composition pharmaceutique selon l'invention comprend de 0,00002 % à 5 %, de préférence de 0,00005 % à 0,1 % et plus préférentiellement encore de 0,0001 % à 0,001 % en poids de peptide selon l'invention et au moins un excipient pharmaceutiquement acceptable.

Selon un mode de réalisation, ladite composition comprend en outre au moins un autre principe dermatologiquement actif.

Plus particulièrement, la composition selon la présente invention comprend en outre au moins un autre principe dermatologiquement actif agissant soit sur le renforcement de la jonction dermo-épidermique, l'adhérence cellules-matrice de la peau, et/ou l'adhérence cellule-cellule et/ou la migration des kératinocytes soit autrement sur la peau selon la nature de ou des agent(s) utilisé(s) tels qu'un agent hydratant, un agent relipidant, un agent surgraissant, un agent exfoliant, un agent kératolytique, un agent antioxydant, un agent apaisant, un agent adoucissant, un agent sédatif, un agent nettoyant, un agent démaquillant, un agent assainissant, un agent antibactérien, un agent antiseptique, un agent antiséborrhéique, un agent éclaircissant, un agent antirides, un agent décongestionnant, un agent revitalisant, un agent activateur du renouvellement cellulaire ou un ou plusieurs filtres solaires.

Les compositions de l'invention doivent se présenter sous une forme dermatologiquement acceptable, c'est-à-dire compatible avec la peau, les poils, les muqueuses et/ou les cheveux.

Ainsi, de manière préférée ladite composition pharmaceutique selon l'invention se présente sous la forme d'une crème, d'une pommade, d'un onguent, d'un masque, d'un sérum, d'un lait, d'une lotion, d'une pâte, d'une mousse, d'un aérosol, d'un stick, d'une poudre, d'une solution, d'une suspension, d'un shampooings, d'un d'après-shampooings, de patchs, d'une solution aqueuse hydroalcoolique ou huileuse, d'une émulsion huile-dans-eau ou eau-dans-huile ou multiple, d'un gel aqueux ou huileux, de fils de suture, de colle chirurgicale, de pansement, d'un produit anhydre liquide, pâteux ou solide, et/ou d'une dispersion d'huile dans une phase aqueuse à l'aide de sphérules, ces sphérules pouvant être des nanoparticules polymériques telles que les nanosphères et les nanocapsules ou des vésicules lipidiques de type ionique et/ou non-ionique.

Préférentiellement, cette composition est destinée à une application topique.

D'éventuels composés additionnels, actifs ou non, pourront être ajoutés à la composition de l'invention et l'homme du métier les choisira de façon à ce que leur adjonction n'altère pas les propriétés avantageuses de ladite composition.

Selon un autre aspect, l'invention concerne une composition cosmétique non-thérapeutique comprenant au moins un peptide selon l'invention.

De manière préférée, ladite composition cosmétique selon l'invention comprend de 0,00002 % à 5 %, de préférence de 0,00005 % à 0,1 % et plus préférentiellement encore de 0,0001 % à 0,001 % en poids de peptide et au moins un excipient cosmétiquement acceptable.

Selon un mode de réalisation, ladite composition comprend en outre au moins un autre principe cosmétiquement actif.

La composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

De manière préférée, ladite composition se présente sous la forme d'une crème, d'une pommade, d'un onguent, d'un masque, d'un sérum, d'un lait, d'une lotion, d'une pâte, d'une mousse, d'un aérosol, d'un stick, d'une poudre, d'une solution, d'une suspension, d'un shampooings, d'un d'après-shampooings, de patchs, d'une solution aqueuse hydroalcoolique ou huileuse, d'une émulsion huile-dans-eau ou eau-dans-huile ou multiple, d'un gel aqueux ou huileux, d'un produit anhydre liquide, pâteux ou solide, et/ou d'une dispersion d'huile dans une phase aqueuse à l'aide de sphérules, ces sphérules pouvant être des nanoparticules polymériques telles que les nanosphères et les nanocapsules ou des vésicules lipidiques de type ionique et/ou non-ionique.

Selon un mode de réalisation, ladite composition est une composition cosmétique de soin du vieillissement cutané tel que les rides, le relâchement, et la perte d'élasticité.

Selon un mode de réalisation, ladite composition est une composition cosmétique de soin de régénération ou de réparation de la peau.

Selon un mode de réalisation, ladite composition est une composition cosmétique de soin du cuir chevelu tel qu'un soin antichute des cheveux.

Selon un mode de réalisation, ladite composition est une composition cosmétique de soin du système pigmentaire de la peau, de l'irrégularité du grain de peau, et/ou de l'altération des phanères.

Selon un autre aspect, l'invention concerne l'utilisation non-thérapeutique d'au moins un peptide selon l'invention dans une composition cosmétique.

Selon un dernier aspect, l'invention concerne un procédé de soin cosmétique de la peau caractérisé en ce qu'il comprend une étape d'application sur la peau d'une composition cosmétique selon l'invention.

De manière préférée, une quantité de 0,2 à 3 mg/cm2 de composition cosmétique est appliqué sur la zone de la peau, de préférence 2 mg/cm2.

De manière préférée, la composition cosmétique selon l'invention est appliquée une à deux fois par jour, de préférence 2 fois par jour, pendant au moins 7 jours, de préférence au moins 15 jours, de manière encore préférée au moins un mois et de manière particulièrement préférée au moins 2 mois. De manière tout particulièrement préférée, la composition cosmétique est appliquée dans le procédé selon l'invention 2 fois par jour pendant au moins 2 mois.

L'invention est définie par les revendications annexées.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
[Fig. 1] La figure 1 illustre les résultats du test d'adhérence cellulaire pour le peptide TALRIRATYGEY (SEQ ID NO : 17) en tant que contrôle positif et des peptides LRIRATYG (SEQ ID NO : 13), LRIRATYGLRIRATYG (SEQ ID NO : 18) et LRIRATYGSGLRIRATYG (SEQ ID NO : 16).
[Fig. 2] La figure 2 illustre les résultats du test d'adhérence cellulaire pour le peptide TALRIRATYGEY (SEQ ID NO : 17) en tant que contrôle positif et des peptides LRIRATYG (SEQ ID NO : 13), LRIRATYGLRIRATYG (SEQ ID NO : 18), CSGLRIRATYGSGC (SEQ ID NO : 15) et LRIRATYGSGLRIRATYG (SEQ ID NO : 16).
[Fig. 3] La figure 3 illustre les résultats du test de fermeture de blessure d'un tapis confluent de kératinocytes primaires pour le peptide TALRIRATYGEY (SEQ ID NO : 17), les peptides LRIRATYG (SEQ ID NO : 13), LRIRATYGLRIRATYG (SEQ ID NO : 18), le peptide cyclique CSGLRIRATYGSGC (SEQ ID NO : 15), un contrôle positif (EGF) et un contrôle négatif (milieu de culture seul). (Les * correspondent à : * p<0,05 ; **p<0,005; ****p<0,0001).
[Fig. 4] La figure 4 illustre les résultats d'un second test de fermeture de blessure d'un tapis confluent de kératinocytes primaires pour le peptide TALRIRATYGEY (SEQ ID NO : 17), le peptide LRIRATYG (SEQ ID NO : 13), le peptide cyclique CSGLRIRATYGSGC (SEQ ID NO : 15), un contrôle positif (EGF) et un contrôle négatif (milieu de culture seul). (Les * correspondent à : * p<0,05 ; **p<0,005; ****p<0,0001).
[Fig. 5] La figure 5 illustre les résultats du test d'adhérence cellulaire pour le peptide TALRIRATYGEY (SEQ ID NO : 17) en tant que contrôle positif et des peptides LRIRATYG (SEQ ID NO : 13), LRIRATYK (SEQ ID NO : 9), et LRIRKTYG (SEQ ID NO : 19).
[Fig. 6] La figure 6 illustre les résultats du test d'adhérence cellulaire pour le peptide TALRIRATYGEY (SEQ ID NO : 17) et les peptides LRIRATYG (SEQ ID NO : 13), LRIRFTYK (SEQ ID NO : 1) et LRIRFTYG (SEQ ID NO : 5).
[Fig. 7] La figure 7 illustre les résultats du test de fermeture de blessure d'un tapis confluent de kératinocytes primaires pour le peptide TALRIRATYGEY (SEQ ID NO : 17) et les peptides LRIRATYG (SEQ ID NO : 13), LRIRFTYG (SEQ ID NO : 5) et LRIRFTYK (SEQ ID NO : 1), un contrôle positif (EGF) et un contrôle négatif (milieu de culture seul). (Les * correspondent à : * p<0,0 5 ; **p<0,005; *** p<0,0005 ; **** p<0,0001).
[Fig. 8] La figure 8 illustre les résultats du test d'adhérence cellulaire pour le peptide LRIRFTYK (SEQ ID NO : 1), et les peptides cycliques CLRIRFTYKC (SEQ ID NO : 2) et CSGLRIRFTYKSGC (SEQ ID NO : 3).
[Fig. 9] La figure 9 illustre les résultats du test de viabilité cellulaire pour les peptides selon l'invention TALRIRATYGEY (SEQ ID NO : 17), LRIRATYG (SEQ ID NO : 13), LRIRFTYK (SEQ ID NO : 1), ainsi que pour le peptide cyclique CLRIRFTYKC (SEQ ID NO : 2).
[Fig. 10] La figure 10 illustre les résultats du test de fermeture de blessure d'un tapis confluent de kératinocytes primaires pour les peptides cycliques selon l'invention CLRIRFTYKC (SEQ ID NO : 2), CSGLRIRFTYKSGC (SEQ ID NO : 3) et pour les peptides LRIRFTYK (SEQ ID NO : 1), LRIRATYG (SEQ ID NO : 13), TALRIRATYGEY (SEQ ID NO : 17), ainsi que pour l'EGF, et le contrôle négatif. (Les * correspondent à : * p<0,05; ***p<0,0005 ; ****p<0,0001 )

### EXEMPLES

### Exemple 1 : Matériel et méthode

### Les cellules utilisées pour l'étude :

- la lignée HT1080 : Ont été utilisées dans un premier temps les cellules provenant de lignées épithéliales, outils couramment utilisés pour les études d'adhérence cellulaire. Les cellules de la lignée HT1080 (fibrosarcoma, Human), American Type Culture Collection CCL-121.
- les kératinocytes humain primaires: Pour le test de viabilité et de migration cellulaires, ont été utilisés des kératinocytes humains normaux fraîchement isolés. Les kératinocytes humains ont été obtenus à partir de biopsie de prépuce (déchet opératoire, Banque de Tissus et cellules, Hôpital Edouard Herriot). Le milieu de culture utilisé au cours des expérimentations a été le milieu défini pour culture de kératinocytes KGM-Gold commercialisé par Lonza Bioscience contenant 0,15 mM de CaCl2, pH 7,2 à 7,4. Les kératinocytes ont été obtenus selon la technique de Boyce et Ham (Cultivation, frozen storage, and clonal growth of normal human epidermal keratinocytes in serum free-media, Tiss Cult. Meth. 1985, 9 :83-93). Les morceaux de peau, après rinçage dans du tampon PBS contenant des antibiotiques, ont été débarrassés du tissu graisseux et découpés en morceaux de 3 mm², puis placés dans une solution stérile de trypsine à 0,25 % dans du PBS pendant 16h à 4°C. La séparation derme / épiderme a été effectuée dans une boîte de Pétri contenant du milieu de culture afin d'arrêter l'action de la trypsine. Les fragments d'épiderme ont été aspirés et refoulés plusieurs fois pour en détacher les cellules basales libres. Après centrifugation, 3.10⁴ cellules vivantes ont été ensemencées par cm² sur des boîtes pour culture de tissus (Corning, Polylabo, France). Les kératinocytes ont été cultivés à 37°C dans un incubateur à CO2 (5 % de CO2, 95% d'air et 98% d'humidité). La sub-culture a eu lieu quand les cellules ont atteint la sub-confluence. Le tapis cellulaire a alors été rincé avec du PBS, puis recouvert d'une solution de Trypsine-EDTA (0,05-0,02 %). Après une courte incubation à 37°C, les cellules se sont détachées, ont été comptées et ré-ensemençées.

### Test de viabilité cellulaire :

L'effet des peptides sur la viabilité cellulaire a été analysé à l'aide d'un test colorimétrique (Cell Proliferation Kit XTT, Roche Diagnostics, Meylan, France) sur les kératinocytes humains normaux provenant de sujets jeunes.

La réaction chimique du test est basée sur la production de NADPH des cellules vivantes permettant la réduction des sels de tretazolium XTT jaunes en sels de formazan orange. La mesure de l'absorbance est effectuée à 490 nm à l'aide d'un lecteur de plaques ELISA-Reader. Les cellules ont été ensemencées dans des plaques 96 puits à raison de 10⁴ cellules par puits (3/condition) dans du milieu de culture KBM-Gold. Après 24h de culture à 37°C en présence de 5% de CO2, les milieux de culture ont été enlevés et remplacés par du milieu KBM-Gold contenant les quantités de peptide indiquées sur les figures. Deux jours après, les milieux ont ensuite été enlevés et remplacés par le réactif du test. Les plaques ont ensuite été placées dans un incubateur à 37°C et la lecture de l'absorbance a été effectuées à 5h.

Des témoins sans peptide ont été réalisés sur la même plaque. Les résultats sont présentés sous la forme du pourcentage de la viabilité des cellules mises en présence du peptide par rapport aux témoins sans peptide. La viabilité cellulaire a été calculée selon la formule :$% viabilit & # xe 9 ; = \left(Abs. cellules avec peptide / Abs. cellules témoins\right) X 100 .$

### Analyse quantitative des propriétés d'adhérence cellulaire des peptides d'intérêt par un test colorimétrique :

### - Préparation des substrats d'adhérence

Une gamme de concentration décroissante des peptides a été réalisée par dilution successive dans du PBS (Phosphate Buffer Saline, KH2PO4 1,54 mM ; Na2HPO4 1,42 mM ; NaCl 131 mM), à partir d'une solution de départ à 1 mg/ml. Ces solutions ont été immédiatement distribuées sur des plaques de culture 96 puits (choix des plaques selon immobilisation) à raison de 100 µl par puit. Les plaques ont ensuite été placées à +4°C pendant 16 à 18h. Les solutions ont ensuite été enlevées et chaque puit a été saturé par une solution de PBS-SAB 1% (sérum albumine bovine). Trois puits supplémentaires sans substrat ont subi le même traitement et ont servi de blanc.

### - Test d'adhérence cellulaire

Les cellules HT1080 ont été détachées des boîtes de culture par une solution de trypsine/EDTA (0,05-0,02%, puis ont été suspendues dans du milieu DMEM sans additifs. Le nombre de cellules semées est de 100 000 cellules par puits.

### - Évaluation du test d'adhérence cellulaire

Après ensemencement des cellules, les plaques ont été placées dans un incubateur à 37° C sous atmosphère 5 % CO2. Après une incubation de 30 à 60 minutes, le milieu d'adhérence a été éliminé et chaque puits lavé avec une solution de PBS stérile afin d'enlever les cellules n'ayant pas adhérées. Les cellules restantes, adhérentes au substrat, ont été fixées par une solution de glutaraldéhyde 1 % dans du PBS, pendant 15 minutes. La solution de glutaraldéhyde a été enlevée et les cellules ont été colorées avec une solution de crystal violet dilué à 1 % dans de l'eau distillée pendant 30 minutes. Après d'importants rinçages, les cellules ont été perméabilisées par une solution de triton à 0,02 % pendant 15 minutes, afin de solubiliser le crystal violet. La lecture de l'absorbance a été effectuée à 570 nm, à l'aide d'un lecteur de plaques (Spectrophotomètre Victor² V, Perkin Elmer). Chaque point expérimental a été réalisé en trois exemplaires. La valeur du blanc, est la moyenne de l'absorbance des 3 puits témoins (SAB). Celle-ci a été soustraite de chacune des valeurs d'absorbance obtenues pour les points expérimentaux. Les moyennes des valeurs d'absorbances pour chacune des conditions ont été calculées et ont été présentées sous forme de courbe, avec en ordonnée, les valeurs moyennes de l'absorbance, et en abscisse, les différentes quantités de substrat immobilisées dans les puits (en µg ou mole).

### Optimisation et vérification de l'immobilisation des peptides dans les plaques

Les différents peptides testés n'ayant pas forcément les mêmes charges, ils s'immobilisent différemment sur les plaques ce qui peut induire des résultats erronés. Ainsi il est nécessaire de tester différents types de plaques et différents tampons d'immobilisation afin de s'assurer de la bonne immobilisation des peptides, surtout dans les études comparatives.

Ainsi les séries de peptides ont été immobilisés dans les conditions décrites dans le test d'adhérence, et après l'immobilisation, la quantité de peptide immobilisée est dosée par une méthode de dosage colorimétrique.

Ce dosage est effectué à l'aide du kit de dosage colorimétrique micro-BCA (PIERCE UPTIMA distribué par Interchim) dans lequel l'intensité de la couleur est directement proportionnelle à la teneur en liaisons peptidiques ayant réduit les ions Cu2+ en ions Cu+, au cours d'une réaction qui est concentration-dépendante. L'acide bicinchoninique est un réactif chromogène spécifique du cuivre (I) formant avec celui-ci un complexe pourpre ayant une absorbance maximale à 562 nm qui est directement propor6onnellle à la concentra6on en protéines. Les protéines sont dosées dans chaque puits par la technique de dosage BCA : 25µL de PBS sont ajoutés dans les puits et sont incubés pendant 30 minutes à 37°C en présence de 200µL de réactif BCA préparé extemporanément selon le protocole fourni dans le kit. La DO est ensuite lue à 562 nm (Spectrophotomètre Victor² V) et le taux de protéines est déterminé par rapport à une gamme étalon réalisée à partir d'un standard protéique commercial de SAB à 2mg/mL.

### Test de fermeture de blessure d'un tapis confluent de kératinocytes primaires

Des plaques 6 puits (Costar) sont recouvertes de collagène I pendant 16h puis rincées avec du PBS. Trois inserts à deux puits Ibidi stériles (Ibidi, référence : 80209) sont déposés dans chacun des puits. 2 x 10⁴ kératinocytes sont déposés dans chaque puits d'insert dans du KBM-Gold. Après deux heures d'incubation dans un air humide à 37°C, composé de 5% de CO2 et de 95% d'air atmosphérique, le milieu de culture est aspiré et les inserts retirés. Du KBM-Gold sans supplément est ajouté dans chacun des puits de la plaque. Les cellules sont ainsi : non traitées (milieu seul), traitées avec du KBM-Gold sans supplément contenant le les peptides étudiés (25 µg/ml), ou traitées avec du KBM-Gold sans supplément contenant l'EGF à 10ng/ml (Peprotech). Les cultures sont stoppées à 12h et 24h, les milieux sont aspirés, et les cellules sont fixées avec du PBS-Paraformaldéhyde à 2% pendant 20 minutes. Après aspiration du fixateur, les cellules sont colorées avec une solution de Crystal Violet à 0,1% dans de l'eau ultra pure pendant 30 minutes. Le Crystal Violet est éliminé et les puits sont rincés à l'eau. Des acquisitions d'images sont effectuées et la quantification des blessures est effectuée grâce au logiciel Adobe Photoshop CS3.

### Exemple 2 : Test d'adhérence - Comparaison entre le peptide TALRIRATYGEY et les peptides LRIRATYG, LRIRATYGLRIRATYG et LRIRATYGSGLRIRATYG

Le test d'adhérence a été réalisé selon le protocole décrit ci-dessus avec des cellules HT1080. Le peptide TALRIRATYGEY (SEQ ID NO : 17) a été utilisé comme peptide contrôle, pour le test des peptides :
- LRIRATYG (SEQ ID NO : 13)
- LRIRATYGLRIRATYG (SEQ ID NO : 18)
- LRIRATYGSGLRIRATYG (SEQ ID NO : 16)

Un dosage protéique a été réalisé pour déterminer la quantité de peptide immobilisée dans chaque puits afin de l'ajuster si nécessaire.

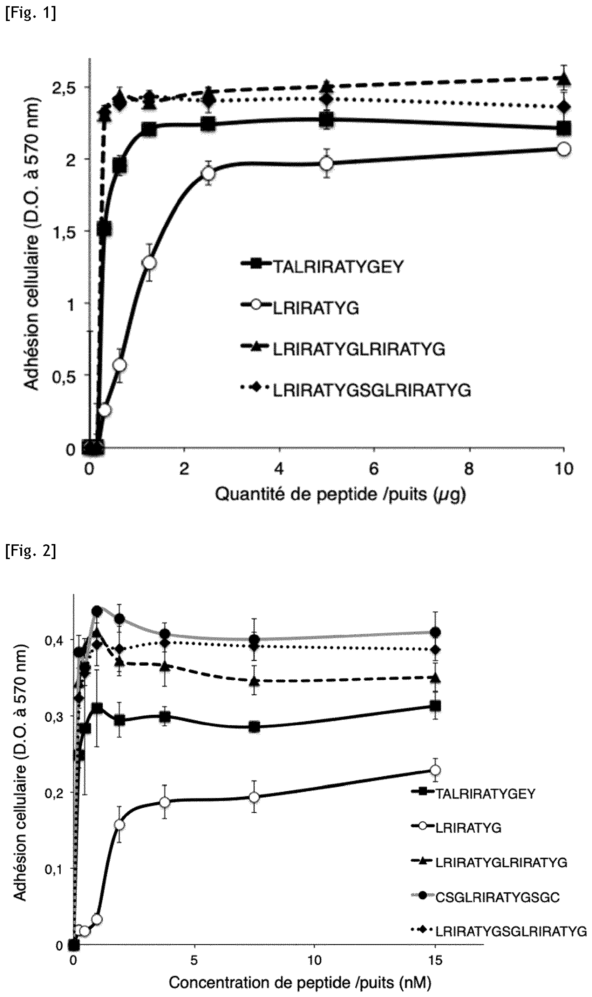

Les résultats sont présentés en figure 1.

Ces résultats montrent que la duplication du motif LRIRATYG (SEQ ID NO : 13) entraîne un doublement d'activité d'induction de l'adhérence à faible concentration.

### Exemple 3 : Test d'adhérence - Comparaison entre le peptide TALRIRATYGEY, les peptides LRIRATYG, LRIRATYGLRIRATYG, LRIRATYGSGLRIRATYG et le peptide cyclique CSGLRIRATYGSGC.

Le test d'adhérence a été réalisé selon le protocole décrit ci-dessus avec des cellules HT1080. Le peptide TALRIRATYGEY (SEQ ID NO : 17) a été utilisé comme peptide contrôle, pour le test des peptides :
- LRIRATYG (SEQ ID NO : 13)
- LRIRATYGLRIRATYG (SEQ ID NO : 18)
- CSGLRIRATYGSGC (SEQ ID NO : 15)
- LRIRATYGSGLRIRATYG (SEQ ID NO : 16)

Un dosage protéique à été réalisé pour déterminer la quantité de peptide immobilisée dans chaque puits afin de l'ajuster si nécessaire.

Les résultats sont présentés en figure 2.

Ces résultats montrent notamment que la cyclisation du motif LRIRATYG (SEQ ID NO : 13), donnant le peptide CSGLRIRATYGSGC (SEQ ID NO : 15) entraîne qu'à faible concentration, l'activité est doublée.

### Exemple 4 : Test de fermeture de blessure d'un tapis confluent de kératinocytes primaires fraichement isolés, avec les peptides TALRIRATYGEY, LRIRATYG, LRIRATYGLRIRATYG, et le peptide cyclique CSGLRIRATYGSGC.

Le test de fermeture de blessure a été réalisé selon le protocole décrit ci-dessus (Exemple 1) avec des kératinocytes primaires humains fraichement isolés. L'Epidermal Growth Factor (EGF) recombinant humain (Peprotech, France) a été utilisé comme contrôle positif et le milieu de culture seul, comme contrôle négatif.

Les peptides testés sont :
- TALRIRATYGEY (SEQ ID NO : 17)
- LRIRATYG (SEQ ID NO : 13)
- LRIRATYGLRIRATYG (SEQ ID NO : 18)
- CSGLRIRATYGSGC (SEQ ID NO : 15)

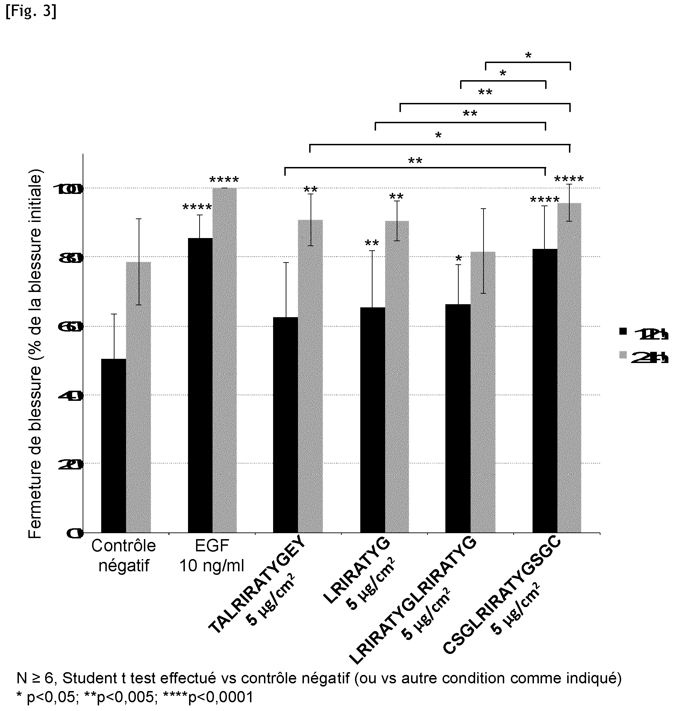

Les résultats sont présentés en figure 3.

Ces résultats montrent que tous les peptides étudiés favorisent significativement la fermeture de blessure à 12h et/ou 24h post-blessure en comparaison du témoin négatif. Le peptide cyclique CSGLRIRATYGSGC (SEQ ID NO : 15) à une activité significativement supérieure à celle des autres peptides et très significativement supérieure à celle du témoin négatif aux deux temps testés.

### Exemple 5 : Test de fermeture de blessure d'un tapis confluent de kératinocytes primaires fraichement isolés avec les peptides TALRIRATYGEY, LRIRATYG, et le peptide cyclique CSGLRIRATYGSGC.

Le test de fermeture de blessure a été réalisé selon le protocole décrit ci-dessus (Exemple 1) avec des kératinocytes primaires humains fraichement isolés. L'Epidermal Growth Factor (EGF) recombinant humain (Peprotech, France) a été utilisé comme contrôle positif et le milieu de culture seul, comme contrôle négatif.

Les peptides testés sont :
- TALRIRATYGEY (SEQ ID NO : 17)
- LRIRATYG (SEQ ID NO : 13)
- CSGLRIRATYGSGC (SEQ ID NO : 15)

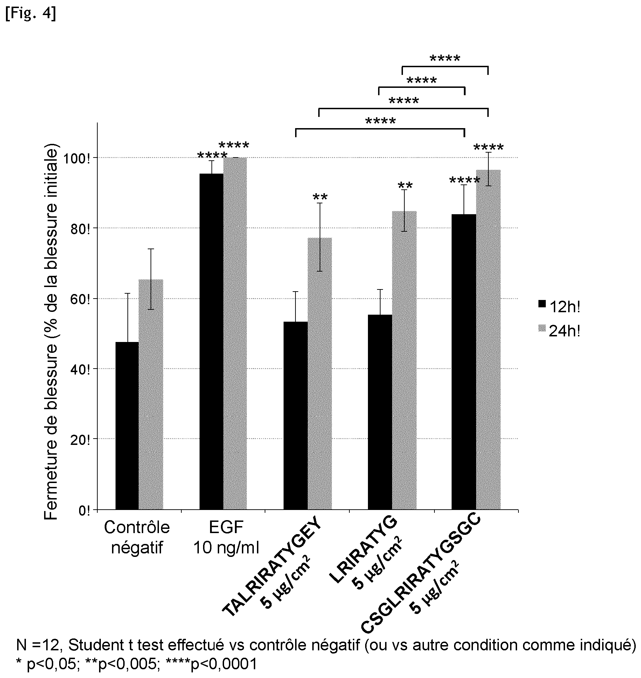

Les résultats sont présentés en figure 4.

Ces résultats montrent que les peptides étudiés, en particulier le peptide cyclique CSGLRIRATYGSGC a une activité significativement supérieure à 12 et 24h en comparaison de celle des peptides TALRIRATYGEY (SEQ ID NO : 17) et LRIRATYG (SEQ ID NO : 13). Dans ce test, on perçoit également que les peptides LRIRATYG et TALRIRATYGEY ont une activité équivalente entre eux à 12 et 24h et que l'activité à 24h est significativement supérieure à celle du témoin négatif. L'activité du peptide cyclique CSGLRIRATYGSGC est significativement supérieure à celle du contrôle négatif.

### Exemple 6 : Test d'adhérence - Comparaison entre le peptide TALRIRATYGEY et les peptides LRIRATYG, LRIRATYK, LRIRKTYG

Le test d'adhérence a été réalisé selon le protocole décrit ci-dessus avec des cellules HT1080. Le peptide TALRIRATYGEY (SEQ ID NO : 17) a été utilisé comme peptide contrôle, pour le test des peptides :
- LRIRATYG (SEQ ID NO : 13)
- LRIRATYK (SEQ ID NO : 9)
- LRIRKTYG (SEQ ID NO : 19)

Un dosage protéique à été réalisé pour déterminer la quantité de peptide immobilisée dans chaque puits afin de l'ajuster si nécessaire.

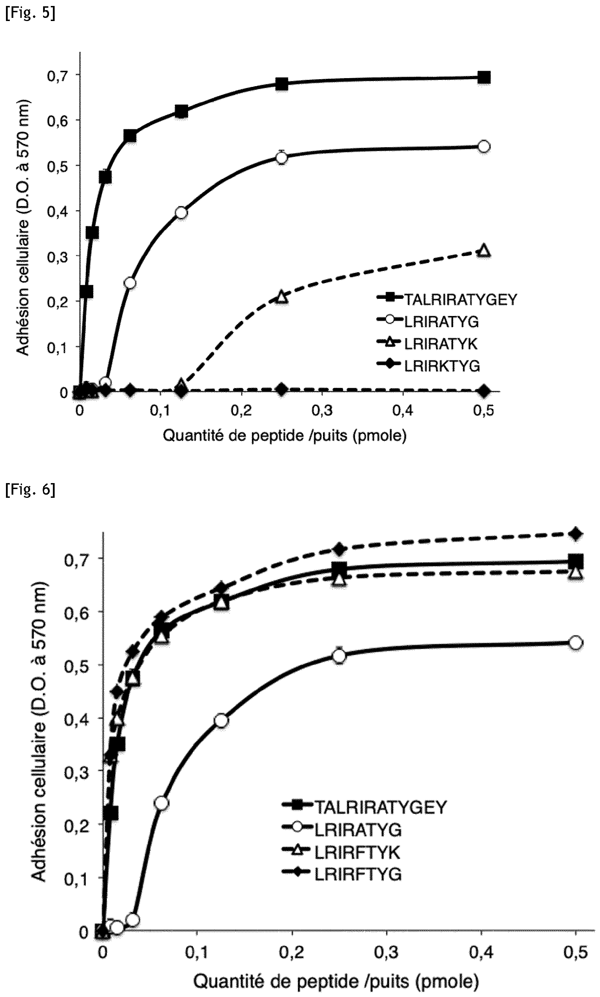

Les résultats sont présentés en figure 5.

Ces résultats montrent que la réponse dépendante de la quantité de peptide immobilisée dans les puits baisse quand la K est positionnée en fin de séquence (LRIRATYK SEQ ID NO : 9) et disparaît quand la K est positionnée en milieu de séquence (LRIRKTYG SEQ ID NO : 19)).

### Exemple 7 : Test d'adhérence - Comparaison entre le peptide TALRIRATYGEY et les peptides LRIRATYG, LRIRFTYK, LRIRFTYG

Le test d'adhérence a été réalisé selon le protocole décrit ci-dessus avec des cellules HT1080.

Le peptide TALRIRATYGEY (SEQ ID NO : 17) a été utilisé comme peptide contrôle pour le test des peptides :
- LRIRATYG (SEQ ID NO : 13)
- LRIRFTYK (SEQ ID NO : 1)
- LRIRFTYG (SEQ ID NO : 5)

Un dosage protéique à été réalisé pour déterminer la quantité de peptide immobilisée dans chaque puits afin de l'ajuster si nécessaire.

Les résultats sont présentés en figure 6.

La réponse dépendante de la quantité de peptide immobilisée dans les puits montre que pour les peptides LRIRFTYK (SEQ ID NO : 1) et LRIRFTYG (SEQ ID NO : 5) les capacités d'adhérence du peptide sont similaires au TALRIRATYGEY contrôle.

### Exemple 8 : Test de fermeture de blessure d'un tapis confluent de kératinocytes primaires fraichement isolés, avec les peptides TALRIRATYGEY, LRIRATYG, LRIRFTYG et LRIRFTYK

Le test de fermeture de blessure a été réalisé selon le protocole décrit ci-dessus (Exemple 1) avec des kératinocytes primaires humains fraichement isolés. Le peptide TALRIRATYGEY (SEQ ID NO : 17) a été utilisé comme peptide contrôle, ainsi que de l'Epidermal Growth Factor (EGF) recombinant humain (Peprotech, France) comme contrôle positif et le milieu de culture seul, comme contrôle négatif.

Les peptides testés sont :
- LRIRATYG (SEQ ID NO : 13)
- LRIRFTYK (SEQ ID NO : 1)
- LRIRFTYG (SEQ ID NO : 5)

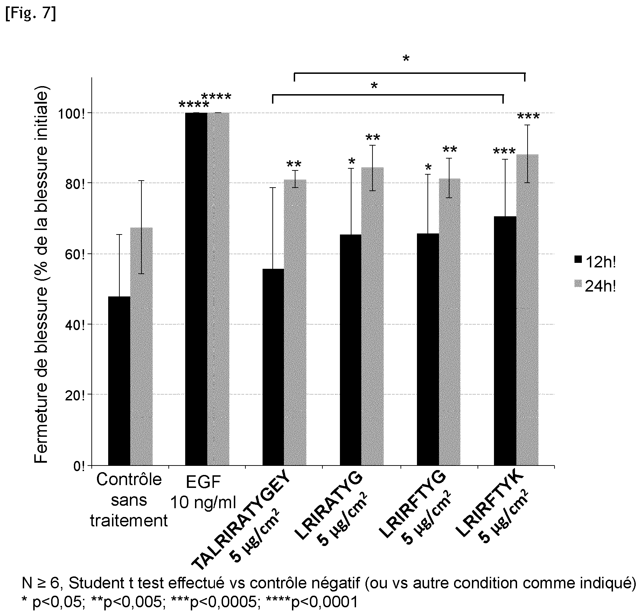

Les résultats sont présentés en figure 7.

Ces résultats montrent que les peptides étudiés ont une activité significativement supérieure à 12 et 24h, voir très significativement supérieure pour le peptide LRIRFTYK (SEQ ID NO : 1), en comparaison au contrôle négatif. Le peptide LRIRFTYK (SEQ ID NO : 1) a une activité significativement supérieure à celle du peptide TALRIRATYGEY (SEQ ID NO : 17) à 12 et 24h post-blessure.

### Exemple 9 : Test d'adhérence pour les peptides cyclisés (CLRIRFTYKC et CSGLRIRFTYKSGC en comparaison au LRIRFTYK.

Les peptides CLRIRFTYKC (SEQ ID NO : 2) et CSGLRIRFTYKSGC (SEQ ID NO : 3) sont des versions cyclisées du peptide LRIRFTYK (SEQ ID NO : 1). Le test d'adhérence a été réalisé selon le protocole décrit ci-dessus (exemple 1) avec des cellules HT1080.

Les trois peptides ont été immobilisés. Des dosages ont été effectués dans les puits afin de déterminer la quantité immobilisée et pour s'assurer que la même quantité de peptide dans les différentes conditions était présente, et l'ajuster si nécessaire.

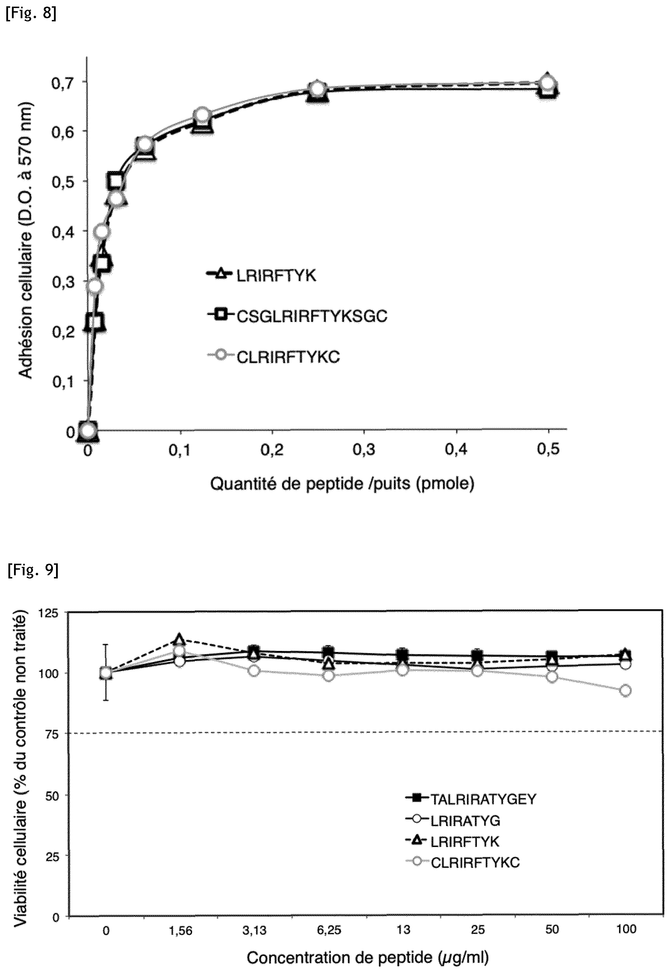

Les résultats sont présentés en figure 8.

La réponse dépendante de la quantité de peptide immobilisé dans les puits montre que les trois peptides ont une activité d'adhésion équivalente. Ainsi le cyclique sans espaceurs (sans SG) est aussi efficace que le cyclique avec espaceurs (SG).

### Exemple 10 : Test de viabilité cellulaire sur des kératinocytes primaires fraichement isolés pour les peptides TALRIRATYGEY, LRIRATYG, LRIRFTYK et CLRIRFTYKC :

Le test de viabilité cellulaire a été réalisé selon le protocole décrit ci-dessus (Exemple 1) avec des kératinocytes primaires fraichement isolés. Le peptide TALRIRATYGEY (SEQ ID NO : 17) a été utilisé comme peptide contrôle.

Les peptides testés sont :
- LRIRATYG (SEQ ID NO : 13)
- LRIRFTYK (SEQ ID NO : 1)
- CLRIRFTYKC (SEQ ID NO : 2)

Les résultats sont présentés en figure 9.

Les résultats, présentés en figure 9, montrent des résultats positifs similaires pour les peptides LRIRATYG (SEQ ID NO : 13), LRIRFTYK (SEQ ID NO : 1)) et CLRIRFTYKC (SEQ ID NO : 2) à ceux du peptide TALRIRATYGEY (SEQ ID NO : 17)

### Exemple 11 : Test de fermeture de blessure d'un tapis confluent de kératinocytes primaires fraichement isolés pour les peptides TALRIRATYGEY, LRIRATYG, LRIRFTYK, CSGLRIRFTYKSGC, et CLRIRFTYKC

Le test de fermeture de blessure a été réalisé selon le protocole décrit ci-dessus (Exemple 1) avec des kératinocytes primaires fraichement isolés. Le peptide TALRIRATYGEY (SEQ ID NO : 17) a été utilisé comme peptide contrôle, l'Epidermal Growth Factor (EGF) recombinant humain (Peprotech, France) comme contrôle positif et le milieu de culture seul, comme contrôle négatif.

Les peptides testés sont :
- LRIRATYG (SEQ ID NO : 13)
- LRIRFTYK (SEQ ID NO : 1)
- CLRIRFTYKC (SEQ ID NO : 2)
- CSGLRIRFTYKSGC (SEQ ID NO : 3)

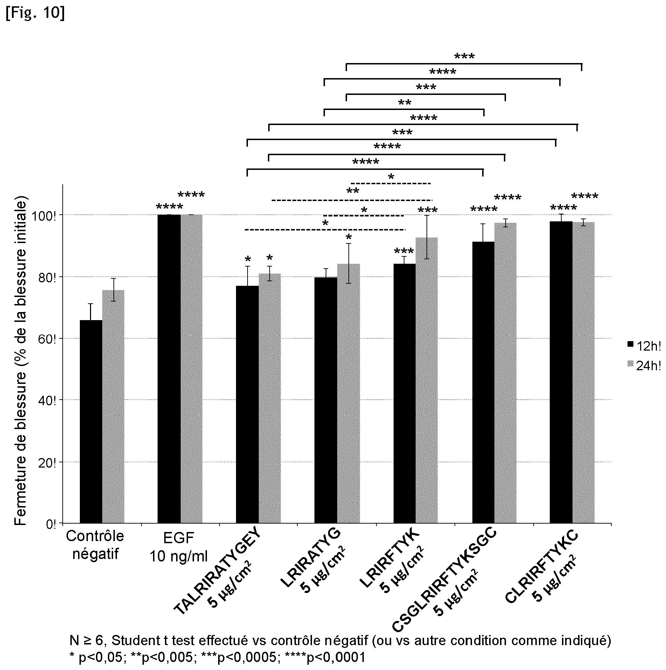

Les résultats sont présentés en figure 10.

Les résultats montrent une activité supérieure significative aux peptides TALRIRATYGEY (SEQ ID NO : 17) et LRIRATYG (SEQ ID NO : 13) pour l'ensemble des peptides étudiés à 12 et 24h. Les peptides ayant les activités les plus importantes sont les peptides, par ordre croissant, LRIRFTYK (SEQ ID NO : 1), CSGLRIRFTYKSGC (SEQ ID NO : 3) et CLRIRFTYKC (SEQ ID NO : 2).

Exemple 8 : Exemples de compositions comprenant les peptides selon l'invention.

### Composition n°1 : Emulsion W/O (eau dans huile)

| Ingrédients | % |
|---|---|
| AQUA (WATER) | QS 100 |
| HEXYL LAURATE | 37.000 |
| GLYCERIN | 5.000 |
| METHYL GLUCOSE ISOSTEARATE | 3.500 |
| DIMETHICONE | 3.000 |
| DISTEARDIMONIUM HECTORITE | 3.000 |
| EUPHORBIA CERIFERA (CANDELILLA) WAX | 1.500 |
| PEG-45/DODECYL GLYCOL COPOLYMER | 1.000 |
| SODIUM SALICYLATE | 0.580 |
| SODIUM BENZOATE | 0.480 |
| MAGNESIUM SULFATE | 0.100 |
| DISODIUM EDTA | 0.100 |
| PEPTIDE LRIRFTYK (SEQ ID N : 1) | 0.0001 |

### Composition N°2 : émulsion O/W (huile dans eau)

| Ingrédients | % |
|---|---|
| AQUA (WATER) | QS 100 |
| CYCLOMETHICONE | 4.000 |
| GLYCERIN | 3.000 |
| HYDROGENATED POLYISOBUTENE | 3.000 |
| PPG-15 STEARYL ETHER | 2.000 |
| ETHYLHEXYL PALMITATE | 2.000 |
| STEARETH-2 | 2.000 |
| STEARETH-21 | 2.000 |
| STEARIC ACID | 1.750 |
| CETYL ALCOHOL | 1.500 |
| SODIUM SALICYLATE | 0.600 |
| POLYACRLAMIDE, C13-14 ISOPARAFFIN, LAURETH-7 | 0.400 |
| XANTHAN GUM | 0.300 |
| SODIUM BENZOATE | 0.200 |
| PEPTIDE CLRIRFTYKC (SEQ ID N : 2) | 0.001 |

### Composition N° 3 : lotion

| Ingrédients | % |
|---|---|
| AQUA (WATER) | QS 100 |
| GLYCERIN | 5.000 |
| SODIUM BENZOATE | 0.700 |
| SODIUM SALICYLATE | 0.550 |
| BIS-PEG-18 METHYL ETHER DIMETHYL SILANE | 0.500 |
| TETRASODIUM EDTA | 0.200 |
| PEPTIDE CSGLRIRFTYKSGC (SEQ ID N : 3) | 0.003 |

### Composition N° 4 : Gel

| Ingrédients | % |
|---|---|
| AQUA (WATER) | QS 100 |
| CYCLOMETHICONE | 4.000 |
| GLYCERIN | 3.000 |
| SODIUM SALICYLATE | 0.600 |
| POLYACRLAMIDE, C13-14 ISOPARAFFIN, LAURETH-7 | 0.500 |
| CARBOMER | 0.500 |
| SODIUM BENZOATE | 0.200 |
| PEPTIDE LRIRFTYKSGLRIRFTYK (SEQ ID N : 4) | 0.003 |

## Revendications

1. Peptide consistant en la séquence : LRIRX₁TYX₂, où :
- X₁ a pour acide aminé A et X₂ a pour acide aminé G ; ou
- X₁ a pour acide aminé F et X₂ est choisi parmi les acides aminés G ou K.

2. Peptide selon la revendication précédente, **caractérisé en ce qu'**il présente la séquence suivante : LRIRFTYK (SEQ ID NO : 1)

3. Peptide consistant en la séquence : CLRIRX₁TYX₂C, où :
- X₁ a pour acide aminé A et X₂ a pour acide aminé G ; ou
- X₁ a pour acide aminé F et X₂ est choisi parmi les acides aminés G ou K.

4. Peptide selon la revendication précédente, **caractérisé en ce qu'**il présente la séquence suivante : CLRIRFTYKC (SEQ ID NO : 2)

5. Peptide consistant en la séquence : CSGLRIRX₁TYX₂SGC, où :
- X₁ a pour acide aminé A et X₂ a pour acide aminé G ; ou
- X₁ a pour acide aminé F et X₂ est choisi parmi les acides aminés G ou K.

6. Peptide selon la revendication précédente, **caractérisé en ce qu'**il présente la séquence suivante : CSGLRIRFTYKSGC (SEQ ID NO : 3).

7. Peptide selon l'une quelconque des revendications 1 à 6 pour utilisation comme médicament.

8. Peptide selon la revendication précédente, pour utilisation comme médicament dans :
- le renforcement de la jonction dermo-épidermique, l'adhérence cellule-cellule et/ou l'adhérence cellule-matrice au niveau de l'épiderme ; et/ou
- le traitement des altérations de la peau induites par des pathologies dermatologiques ; et/ou
- le traitement des altérations de la peau fragilisée par des traitements cosmétiques ou thérapeutiques ; et/ou
- le traitement curatif ou préventif du vieillissement cutané tel que les rides, le relâchement, la perte d'élasticité, la moins bonne cicatrisation, de la xérose sénile, des modifications du système pigmentaire de la peau, de l'appauvrissement du réseau vasculaire de la peau, de l'irrégularité du grain de peau, de l'atrophie cutanée et de l'altération des phanères.

9. Composition cosmétique non-thérapeutique **caractérisée en ce qu'**elle comprend au moins un peptide selon l'une quelconque des revendications 1 à 6.

10. Composition cosmétique selon la revendication précédente **caractérisée en ce qu'**elle comprend de 0,00002 % à 5 %, de préférence de 0,00005 % à 0,1 % et plus préférentiellement encore de 0,0001 % à 0,001 % en poids de peptide et au moins un excipient cosmétiquement acceptable.

## Patentansprüche

1. Peptid, bestehend aus der Sequenz: LRIRX₁TYX₂, wobei:
- X₁ die Aminosäure A aufweist und X₂ die Aminosäure G aufweist; oder
- X₁ die Aminosäure F aufweist und X₂ aus den Aminosäuren G oder K ausgewählt ist.

2. Peptid nach dem vorstehenden Anspruch,
**dadurch gekennzeichnet, dass** es die folgende Sequenz aufweist: LRIRFTYK (SEQ ID NO: 1)

3. Peptid, bestehend aus der Sequenz: CLRIRX₁TYX₂C, wobei:
- X₁ die Aminosäure A aufweist und X₂ die Aminosäure G aufweist; oder
- X₁ die Aminosäure F aufweist und X₂ aus den Aminosäuren G oder K ausgewählt ist.

4. Peptid nach dem vorstehenden Anspruch,
**dadurch gekennzeichnet, dass** es die folgende Sequenz aufweist: CLRIRFTYKC (SEQ ID NO: 2)

5. Peptid, bestehend aus der Sequenz: CSGLRIRX₁TYX₂SGC, wobei:
- X₁ die Aminosäure A aufweist und X₂ die Aminosäure G aufweist; oder
- X₁ die Aminosäure F aufweist und X₂ aus den Aminosäuren G oder K ausgewählt ist.

6. Peptid nach dem vorstehenden Anspruch,
**dadurch gekennzeichnet, dass** es die folgende Sequenz aufweist: CSGLRIRFTYKSGC (SEQ ID NO: 3).

7. Peptid nach einem der Ansprüche 1 bis 6 zur Verwendung als Arzneimittel.

8. Peptid nach dem vorstehenden Anspruch zur Verwendung als Arzneimittel bei:
- der Verstärkung der dermoepidermalen Junktionszone, der Zell-Zell-Adhäsion und/oder der Zell-Matrix-Adhäsion auf der Ebene der Epidermis; und/oder
- der Behandlung von durch dermatologische Erkrankungen induzierten Hautveränderungen; und/oder
- der Behandlung der Veränderungen der durch kosmetische oder therapeutische Behandlungen geschwächten Haut; und/oder
- der heilenden oder präventiven Behandlung der Hautalterung wie Falten, Erschlaffung, Elastizitätsverlust, verschlechterte Wundheilung, senile Xerose, Veränderungen des Pigmentsystems der Haut, Verarmung des Gefäßnetzes der Haut, Unregelmäßigkeit des Hautbildes, Hautatrophie und Veränderung der Hautanhangsgebilde.

9. Nicht therapeutische kosmetische Zusammensetzung,
**dadurch gekennzeichnet, dass** sie mindestens ein Peptid nach einem der Ansprüche 1 bis 6 umfasst.

10. Kosmetische Zusammensetzung nach dem vorstehenden Anspruch,
**dadurch gekennzeichnet, dass** sie zu 0,00002 Gew.-% bis 5 Gew.-%, vorzugsweise 0,00005 Gew.-% bis 0,1 Gew.-% und noch mehr bevorzugt 0,0001 Gew.-% bis 0,001 Gew.-% Peptid und mindestens einen kosmetisch verträglichen Hilfsstoff umfasst.

## Claims

1. Peptide consisting of the sequence: LRIRX₁TYX₂, where:
- X₁ has amino acid A and X₂ has amino acid G; or
- X₁ has amino acid F and X₂ is selected from the amino acids G or K.

2. Peptide according to the preceding claim, **characterized in that** it has the following sequence: LRIRFTYK (SEQ ID NO: 1)

3. Peptide consisting of the sequence: CLRIRX₁TYX₂C, where:
- X₁ has amino acid A and X₂ has amino acid G; or
- X₁ has amino acid F and X₂ is selected from the amino acids G or K.

4. Peptide according to the preceding claim, **characterized in that** it has the following sequence: CLRIRFTYKC (SEQ ID NO: 2)

5. Peptide consisting of the sequence: CSGLRIRX₁TYX₂SGC, where:
- X₁ has amino acid A and X₂ has amino acid G; or
- X₁ has amino acid F and X₂ is selected from the amino acids G or K.

6. Peptide according to the preceding claim, **characterized in that** it has the following sequence: CSGLRIRFTYKSGC (SEQ ID NO: 3).

7. Peptide according to any of claims 1 to 6 for use as a medication.

8. Peptide according to the preceding claim, for use as a medication in:
- the reinforcement of the dermal-epidermal junction, cell-cell adhesion and/or cell-matrix adhesion in the epidermis; and/or
- the treatment of impairments of the skin induced by dermatological pathologies; and/or
- the treatment of impairments of the skin weakened by cosmetic or therapeutic treatments; and/or
- the curative or preventive treatment of skin aging such as wrinkles, loosening, loss of elasticity, reduced healing, senile xerosis, changes in the pigment system of the skin, depletion of the vascular network of the skin, irregularity of the skin texture, skin atrophy and alteration of the skin appendages.

9. Non-therapeutic cosmetic composition **characterized in that** it comprises at least one peptide according to any of claims 1 to 6.

10. Cosmetic composition according to the preceding claim,
**characterized in that** it comprises from 0.00002% to 5%, preferably from 0.00005% to 0.1% and even more preferentially from 0.0001% to 0.001% by weight of the peptide and at least one cosmetically acceptable excipient.
